# EUROPEAN PATENT APPLICATION

(11) **EP 4 831 197 A1**
(43) Date of publication of application: **28.10.2026**
(21) Application number: 23961900.0
(22) Date of filing: 21.12.2023
(51) Int. Cl.: C07K 16/28, C07K 19/00, A61K 47/62, A61K 39/00, A61K 39/39, A61P 35/00

(54) **CD276 BINDING MINIPROTEIN, MUTANT THEREOF, AND USE THEREOF**

(71) Applicant: Westlake Laboratory of Life Sciences and Biomedicine, Hangzhou, Zhejiang 310024 (CN)
(72) Inventor: CAO, Longxing, Hangzhou, Zhejiang 310024 (CN); XIE, Qi, Hangzhou, Zhejiang 310024 (CN); JIN, Qihan, Hangzhou, Zhejiang 310024 (CN); XIA, Zhen, Hangzhou, Zhejiang 310024 (CN)
(74) Representative: Grünecker Patent- und Rechtsanwälte PartG mbB
(86) International application number: PCT/CN2023/140615
(87) International publication number: WO 2025/129565

(57) **Abstract**

Provided are a CD276 binding miniprotein, a mutant thereof, and a use thereof. Further provided are a chimeric antigen receptor comprising the CD276 binding miniprotein or the mutant thereof as an extracellular antigen binding domain, and a chimeric antigen receptor T cell. The binding affinity of a CD276 binding protein mutant HM9 for CD276 reaches 36.4 pM, and the binding protein has a small molecular weight, good stability and high affinity, and can be massively expressed in Escherichia coli. Compared with traditional scFC-based CAR-T treatment strategies, the constructed CAR-T cell has higher tumor killing activity, a lower exhaustion level, and a faster division and expansion capability.

## Description

### TECHNICAL FIELD

The present application belongs to the field of tumor immunotherapy drugs, and specifically relates to a newly designed CD276 binding miniprotein, mutant thereof, and use thereof.

### BACKGROUND ART

B7 homolog 3 (B7-H3), more commonly known as CD276, is a co-stimulatory/co-inhibitory regulatory protein that plays a dual role in the immune system and is also an immune checkpoint-associated protein. It was first cloned and identified in 2001 from a cDNA library derived from human dendritic cells. The human CD276 gene is located on chromosome 15 and is mostly expressed as a membrane protein, specifically a type I transmembrane protein containing 316 amino acids, consisting of an extracellular domain, a transmembrane domain, and a short intracellular domain. Studies have shown that CD276 expression is abnormally elevated in tumor cells, leading to increased adhesion of cell adhesion proteins and enhanced tumor cell migration and invasion. Furthermore, CD276 can increase NF-κB activity, resulting in elevated VEGF and IL-8 expression, further promoting tumor-associated angiogenesis and tumor invasion. Because CD276 is abnormally highly expressed in various cancer cells, including medulloblastoma and glioblastoma, and its distribution in normal tissues is limited, it is an effective and safe target. Currently, various B7-H3-targeted immunotherapy strategies, including monoclonal antibodies, bispecific antibodies, ADCs, and CAR-T, have been developed and tested in clinical trials, demonstrating promising application prospects and representing a highly attractive target. Designing and developing more efficient protein drugs and immunotherapy strategies targeting CD276 holds immense value. Protein design, based on biophysical and biochemical principles, involves designing the amino acid sequence of proteins to enable them to spontaneously fold into desired novel three-dimensional structures with new functions. The design of functional proteins, especially novel binding proteins with high affinity and specificity directing to any naturally occurring protein, has crucial applications in diagnosis, treatment, and prevention of diseases. Compared to protein drugs represented by monoclonal antibodies, artificially designed binding proteins offer significant advantages in the following aspects: small molecular weight, good tissue penetration, strong chemical activity, good stability, and ease of industrialization. They can be rapidly designed to target specific epitopes and their binding mechanisms can be precisely controlled. Designing binding proteins entirely based on the three-dimensional structural information of the target protein has become possible, and new protein design algorithms and tools provide new tools and research models for basic protein research and protein drug development. Developing protein drugs and immunotherapies targeting CD276 based on the latest protein design algorithms has significant application prospects and value.

CAR-T therapy is considered another revolutionary breakthrough in tumor immunotherapy after immune checkpoint inhibitors, and has already achieved great success in the field of hematologic malignancies. CAR-T therapy involves collecting and isolating T cells from the patient's blood, and then bioengineering them to express chimeric antigen receptors (CARs) that specifically recognize tumor surface antigens, thereby enhancing their targeting and killing ability against cancer cells. The extracellular domain of the key CAR-T receptor is mainly composed of single-chain variable fragments (scFvs) of monoclonal antibodies responsible for recognizing and binding antigens. scFvs are composed of the light chain (VL) and heavy chain (VH) of the monoclonal antibody linked by a polypeptide, retaining the antibody's specificity and affinity for the antigen. However, the heavy and light chains of an scFv are prone to mis-aggregation and folding, resulting in poor structural stability. This not only affects the ability of T cells to recognize antigen cells but can also induce antigen-independent CAR aggregation and T cell activation, leading to early-stage exhausted T cells. This significantly reduces the therapeutic efficacy of CAR-T therapy. Given the abnormally high expression of CD276 in many solid tumor cells, the de novo design and development of CD276-targeting binding proteins to develop next-generation CAR-T therapy strategies targeting CD276 has significant research and clinical application value.

### SUMMARY OF THE INVENTION

To address the above technical problems, in one aspect, this invention provides a CD276 binding miniprotein or a mutant thereof,
wherein, the CD276 binding miniprotein has an amino acid sequence set forth in SEQ ID No.: 1, and
the mutant thereof is selected from:
   i) a mutant with an amino acid sequence set forth in SEQ ID No.: 2;
   ii) a single-site mutant of SEQ ID No.: 1,
with the amino acid at position 1 mutated to any one of A, D, E, F, G, H, I, L, M, N, P, R, T, V, W, and Y; or
with the amino acid at position 2 mutated to any one of A, D, E, F, G, H, K, L, M, N, P, Q, R, S, T, V, and W; or
with the amino acid at position 3 mutated to any one of A, D, E, F, G, H, I, K, L, M, N, P, Q, T, V, W, and Y; or
with the amino acid at position 4 mutated to any one of A, D, F, G, H, I, K, L, M, N, P, Q, R, S, T, V, W, and Y; or
with the amino acid at position 5 mutated to any one of A, D, E, H, I, N, Q, S, T, and V; or
with the amino acid at position 6 mutated to any one of F, L, M, W, and Y; or
with the amino acid at position 7 mutated to any one of A, D, E, F, G, H, I, K, L, M, N, Q, R, S, T, V, W, and Y; or
with the amino acid at position 8 mutated to any one of I, M, and V;
with the amino acid at position 9 mutated to any one of F, H, I, L, and Y; or
with the amino acid at position 10 mutated to any one of A, D, E, F, H, I, L, M, Q, S, T, V, W, and Y; or
with the amino acid at position 11 mutated to any one of A, H, K, N, Q, R, S, T, V, and Y; or
with the amino acid at position 12 mutated to T; or
with the amino acid at position 13 mutated to any one of A, F, G, S, and T; or
with the amino acid at position 14 mutated to any one of A, E, F, G, H, I, K, L, M, N, Q, R, S, T, V, W, and Y; or
with the amino acid at position 1 mutated to any one of A, D, G, H, N, S, T, and V; or
with the amino acid at position 16 mutated to L; or
with the amino acid at position 17 mutated to any one of E, F, I, L, M, T, and V; or
with the amino acid at position 18 mutated to any one of A, H, K, N, Q, R, and T; or
with the amino acid at position 19 mutated to any one of A, F, G, H, I, L, M, N, Q, R, S, T, and V; or
with the amino acid at position 20 mutated to any one of A, D, E, G, H, K, N, Q, R, S, and T; or
with the amino acid at position 21 mutated to any one of D, E, K, N, and Q; or
with the amino acid at position 22 mutated to any one of A, F, H, K, L, M, N, Q, R, S, T, V, and Y; or
with the amino acid at position 23 mutated to any one of A, D, E, F, G, H, I, K, L, M, N, P, Q, R, S, T, and Y; or
with the amino acid at position 24 mutated to any one of L, M, V, and W; or
with the amino acid at position 25 mutated to any one of L, and M; or
with the amino acid at position 26 mutated to any one of A, C, D, E, F, H, I, K, L, M, N, P, Q, R, S, T, V, W, and Y; or
with the amino acid at position 27 mutated to any one of H, I, P, and V; or
with the amino acid at position 28 mutated to any one of A, C, I, L, M, S, and V; or
with the amino acid at position 29 mutated to any one of A, C, E, F, G, H, I, K, L, M, N, Q, R, S, T, V, W, and Y; or
with the amino acid at position 30 mutated to any one of A, D, E, F, G, H, K, M, N, Q, R, and W;
with the amino acid at position 31 mutated to any one of G, and S; or
with the amino acid at position 32 mutated to any one of A, C, F, I, L, M, S, T, V, and Y; or
with the amino acid at position 33 mutated to any one of A, C, D, E, G, H, I, K, L, M, N, Q, R, S, T, and Y; or
with the amino acid at position 34 mutated to any one of F, H, M, T, W, and Y; or
with the amino acid at position 35 mutated to any one of A, F, H, M, N, Q, and Y; or
with the amino acid at position 36 mutated to any one of C, D, E, F, H, I, K, L, M, N, R, S, V, W, and Y; or
with the amino acid at position 37 mutated to any one of A, D, E, F, G, H, I, K, L, M, N, Q, R, S, T, W, and Y; or
with the amino acid at position 38 mutated to any one of A, K, M, Q, R, S, and V; or
with the amino acid at position 39 mutated to any one of H, K, N, R, S, T, V, and Y;
with the amino acid at position 40 mutated to any one of A, C, D, E, G, H, K, M, N, Q, R, S, and Y; or
with the amino acid at position 41 mutated to any one of A, D, E, H, N, Q, S, and W; or
with the amino acid at position 42 mutated to any one of A, D, E, F, G, H, I, K, L, M, N, P, Q, R, S, T, V, W, and Y; or
with the amino acid at position 43 mutated to any one of A, G, I, K, N, R, S, T, V, and Y; or
with the amino acid at position 44 mutated to any one of A, G, and S; or
with the amino acid at position 45 mutated to any one of the following: A, D, F, G, H, K, L, M, N, Q, R, S, V, W, and Y; or
with the amino acid at position 46 mutated to any one of the following: A, D, E, F, H, I, K, L, M, N, P, Q, R, S, T, V, W, and Y; or
with the amino acid at position 47 mutated to any one of the following: I, L, M, Q, and V; or
with the amino acid at position 48 mutated to any one of the following: A, C, D, E, F, G, H, I, K, L, M, N, Q, R, S, T, V, W, and Y; or
with the amino acid at position 49 mutated to any one of the following: A, D, E, F, G, H, I, K, L, M, N, Q, R, S, T, V, W, and Y; or
with the amino acid at position 50 mutated to any one of the following: A, D, E, F, G, H, I, K, L, M, N, Q, R, S, T, V, W, and Y; or
with the amino acid at position 51 mutated to any one of A, C, G, I, L, M, S, T, and V; or
with the amino acid at position 52 mutated to any one of A, C, D, E, F, G, H, I, K, L, M, N, Q, R, S, T, V, W, and Y; or
with the amino acid at position 53 mutated to any one of A, C, D, E, F, G, H, I, K, L, M, N, Q, R, S, T, V, W, and Y; or
with the amino acid at position 54 mutated to any one of A, E, F, H, I, K, L, M, N, Q, R, S, T, V, W, and Y; or
with the amino acid at position 55 mutated to any one of F, I, L, M, S, and T.

Preferably, the CD276 binding miniprotein mutant has an amino acid sequence of SEQ ID No.: 2. In another aspect, the present invention provides an isolated nucleic acid comprising a nucleic acid sequence encoding the aforementioned CD276 binding miniprotein or the mutant thereof.

In yet another aspect, the present invention provides a vector comprising the aforementioned isolated nucleic acid.

In a further aspect, the present invention provides a chimeric antigen receptor comprising, from N-terminus to C-terminus:
(a) a signal peptide;
(b) an extracellular antigen-binding domain comprising the above CD276 binding miniprotein or the mutant thereof or composed of the above CD276 binding miniprotein or the mutant thereof;
(c) a hinge region;
(d) a transmembrane domain;
(e) an intracellular co-stimulatory signal transduction domain; and
(f) an intracellular stimulatory signal transduction domain.

In a specific embodiment, the signal peptide is derived from human CD8α and has an amino acid sequence of SEQ ID No.: 4; the hinge region is derived from human CD8α and has an amino acid sequence of SEQ ID No.: 5; the transmembrane domain is derived from human CD8α and has an amino acid sequence of SEQ ID No.: 6; the intracellular co-stimulatory signal transduction domain is derived from human 4-1BB and has an amino acid sequence of SEQ ID No.: 7; and the intracellular stimulatory signal transduction domain is derived from human CD3ζ and has an amino acid sequence of SEQ ID No.: 8.

In a specific embodiment, the chimeric antigen receptor has an amino acid sequence of SEQ ID No.: 3.

In a further aspect, the present invention provides an engineered immune effector cell comprising the aforementioned chimeric antigen receptor.

In a specific embodiment, the immune effector cell is a T cell, NK cell, or macrophage.

In a specific embodiment, the engineered immune effector cell is a CAR-T cell.

In yet another aspect, the present invention provides a pharmaceutical composition comprising the aforementioned engineered immune effector cell and a pharmaceutically acceptable carrier. In yet another aspect, the present invention provides an antibody comprising the aforementioned CD276 binding miniprotein or the mutant thereof.

In yet another aspect, the present invention provides the use of the aforementioned CD276 binding miniprotein or the mutant thereof in the preparation of a monoclonal antibody, a bispecific antibody, an antibody-drug conjugate, or an engineered immune effector cell.

In yet another aspect, the present invention provides the use of the aforementioned CD276 binding miniprotein or a mutant thereof, the aforementioned engineered immune effector cell, the aforementioned pharmaceutical composition, or the aforementioned antibody in the preparation of an antitumor drug.

In a specific embodiment, the tumor is selected from glioblastoma (GBM).

### BENEFICIAL EFFECTS

In the present application, a binding protein targeting the CD276 protein that is abnormally highly expressed on the surface of various solid tumor cancer cells was precisely designed. It has been optimized for its affinity and stability, and its biochemical properties were further identified. A binding protein, HM9, was precisely designed for CD276 (B7-H3), and its affinity was optimized using a yeast surface display method, ultimately achieving a binding affinity of 36.4 pM for CD276. This binding protein has a small molecular weight, good stability, high affinity, and can be expressed in large quantities in *E. coli*.

Furthermore, based on the artificially designed CD276-targeting binding protein HM9, CAR-T cells targeting CD276 were constructed. The killing, proliferation, and exhaustion effects of these CAR-T cells on several tumor cell lines highly expressing CD276 were verified at the cellular and animal levels, and the effects were compared with CAR-T cells constructed by using an scFv targeting CD276. Compared to traditional scFv-based CAR-T therapy strategies, CAR-T cells based on the artificially designed binding protein HM9 have higher tumor-killing activity, lower exhaustion levels, and faster division and expansion capabilities.

### BRIEF DESCRIPTION OF THE FIGURES

Figure 1: Flow cytometry enrichment analysis heatmap of site saturation mutagenesis library of CD276_mb.
Figure 2: PAGE gel image of CD276_mb and HM9 expressed in *E. coli* BL21(DE3) during the protein purification process. In the figure, M represents Blue Plus V Protein Marker (DM141-01, TransGen), O represents whole cell lysate, S represents supernatant, F represents flow-through, W represents washing solution, E represents elution solution, and A represents protein solution purified by molecular sieve chromatography.
Figure 3: Results of purification of CD276_mb and HM9 using a Superdex 75 10/300 GL molecular sieve chromatography column. Orange represents CD276_mb, and blue represents HM9.
Figure 4: A graph showing the affinity between CD276_mb and CD276 determined using BLI.
Figure 5: A graph showing the affinity between HM9 and CD276 determined using BLI.
Figure 6: A diagram showing the design of CD276 mb CAR-T cells.
Figure 7 shows the killing, T cell proliferation, and exhaustion effects of the HM9 CAR-T cells on different tumor cells determined by flow cytometry analysis.
Figure 8: Flowchart of CAR-T therapy in orthotopic tumor-bearing mice.
Figure 9: A graph showing the effects of HM9-4-1BB CAR-T therapy on survival time (A) and tumor signaling (B) in orthotopic tumor-bearing mice.

### DETAILED EMBODIMENTS

The technical solutions of the present application are described in detail below through specific embodiments to enable those skilled in the art to better understand the technical solutions of the present application. However, the scope of the present application is not limited thereto, any equivalent and replacement that those skilled in the art can make based on the present application also fall within the scope of the present application.

### Terms

In the present application, the CD276 binding miniprotein can be also represented as: CD276_mb.

**Example 1**: Flow Cytometry Enrichment Analysis Heatmap of the site saturation mutagenesis (SSM) library of CD276_mb (SEQ ID No.: 1)

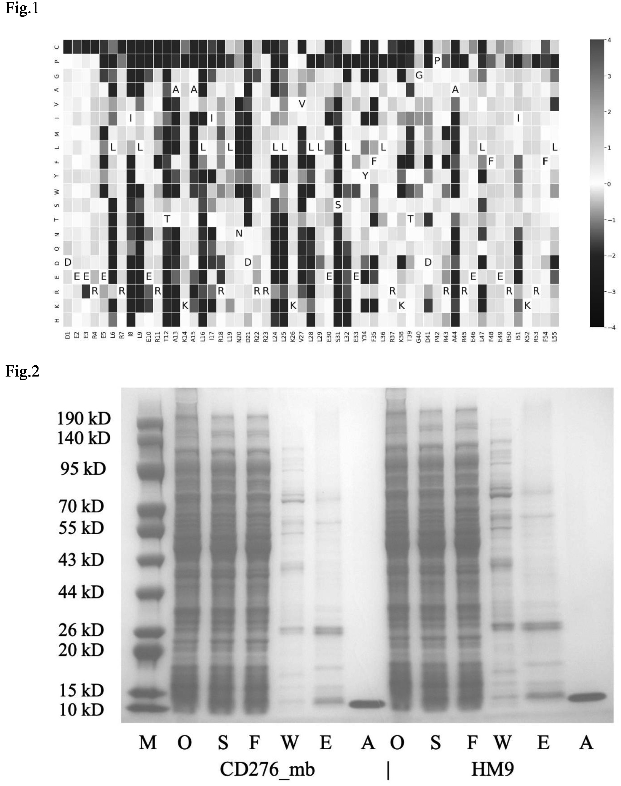

The SSM library of CD276_mb was constructed into the pETCON3 plasmid, the N-terminus of which was fused with the Aga1p-Factor Xa site-HA-GS Linker (SEQ ID No.: 9), and the C-terminus of which was fused with the cMyc tag (SEQ ID No.: 10). Subsequently, the constructed plasmid was electroporated into the *Saccharomyces cerevisiae* EBY100 strain and cultured in SDCAA medium supplemented with 2% (w/v) glucose for 48 h. Yeast cells were transferred to SGCAA medium supplemented with 0.2% (w/v) glucose such that an initial cell density reached 0.5 × 10⁷ cells/ml and the cells were induced at 30°C and 200 RPM for 16 h. Cells were collected by centrifugation at 4000g for 1 min, washed with PBS supplemented with 0.1% (w/v) BSA, incubated with biotinylated CD276 for 30 min, washed again, and incubated with anti-cMyc fluorescein isothiocyanate (FITC, 130-116-653, Miltenyi Biotech) and streptavidin-phycoerythrin (SAPE, SA10044, Thermo Fisher) for 30 min. The cells were then washed and analyzed by flow cytometry. The concentration of biotinylated CD276 was gradually decreased from 1 µM to 10 nM. Deep sequencing was performed on the results of each round of sorting to analyze which point mutations gradually enriched. Gradually enriched mutations were shown in red, and gradually decreasing mutations were shown in blue. The analysis heatmap is shown in Figure 1.

As can be seen from Figure 1, some single amino acid mutations were significantly enriched, meaning that these mutations may enhance the affinity between CD276_mb and CD276. These point mutations include, but are not limited to, the following:
the amino acid at position 1 is mutated to any one of A, D, E, F, G, H, I, L, M, N, P, R, T, V, W, and Y; or
the amino acid at position 2 is mutated to any one of A, D, E, F, G, H, K, L, M, N, P, Q, R, S, T, V, and W; or
the amino acid at position 3 is mutated to any one of A, D, E, F, G, H, I, K, L, M, N, P, Q, T, V, W, and Y; or
the amino acid at position 4 is mutated to any one of A, D, F, G, H, I, K, L, M, N, P, Q, R, S, T, V, W, and Y; or
the amino acid at position 5 is mutated to any one of A, D, E, H, I, N, Q, S, T, and V; or
the amino acid at position 6 is mutated to any one of F, L, M, W, and Y; or
the amino acid at position 7 is mutated to any one of A, D, E, F, G, H, I, K, L, M, N, Q, R, S, T, V, W, and Y; or
the amino acid at position 8 is mutated to any one of I, M, and V;
the amino acid at position 9 is mutated to any one of F, H, I, L, and Y; or
the amino acid at position 10 is mutated to any one of A, D, E, F, H, I, L, M, Q, S, T, V, W, and Y; or
the amino acid at position 11 is mutated to any one of A, H, K, N, Q, R, S, T, V, and Y; or
the amino acid at position 12 is mutated to T; or
the amino acid at position 13 is mutated to any one of A, F, G, S, and T; or
the amino acid at position 14 is mutated to any one of A, E, F, G, H, I, K, L, M, N, Q, R, S, T, V, W, and Y; or
the amino acid at position 15 is mutated to any one of A, D, G, H, N, S, T, and V; or
the amino acid at position 16 is mutated to L; or
the amino acid at position 17 is mutated to any one of E, F, I, L, M, T, and V; or
the amino acid at position 18 is mutated to any one of A, H, K, N, Q, R, and T; or
the amino acid at position 19 is mutated to any one of A, F, G, H, I, L, M, N, Q, R, S, T, and V; or
the amino acid at position 20 is mutated to any one of A, D, E, G, H, K, N, Q, R, S, and T; or
the amino acid at position 21 is mutated to any one of D, E, K, N, and Q; or
the amino acid at position 22 is mutated to any one of A, F, H, K, L, M, N, Q, R, S, T, V, and Y; or
the amino acid at position 23 is mutated to any one of A, D, E, F, G, H, I, K, L, M, N, P, Q, R, S, T, and Y; or
the amino acid at position 24 is mutated to any one of L, M, V, and W; or
the amino acid at position 25 is mutated to any one of L, and M; or
the amino acid at position 26 is mutated to any one of A, C, D, E, F, H, I, K, L, M, N, P, Q, R, S, T, V, W, and Y; or
the amino acid at position 27 is mutated to any one of H, I, P, and V; or
the amino acid at position 28 is mutated to any one of A, C, I, L, M, S, and V; or
the amino acid at position 29 is mutated to any one of A, C, E, F, G, H, I, K, L, M, N, Q, R, S, T, V, W, and Y; or
the amino acid at position 30 is mutated to any one of A, D, E, F, G, H, K, M, N, Q, R, and W;
the amino acid at position 31 is mutated to any one of G, and S; or
the amino acid at position 32 is mutated to any one of A, C, F, I, L, M, S, T, V, and Y; or
the amino acid at position 33 is mutated to any one of A, C, D, E, G, H, I, K, L, M, N, Q, R, S, T, and Y; or
the amino acid at position 34 is mutated to any one of F, H, M, T, W, and Y; or
the amino acid at position 35 is mutated to any one of A, F, H, M, N, Q, and Y; or
the amino acid at position 36 is mutated to any one of C, D, E, F, H, I, K, L, M, N, R, S, V, W, and Y; or
the amino acid at position 37 is mutated to any one of A, D, E, F, G, H, I, K, L, M, N, Q, R, S, T, W, and Y; or
The amino acid at position 38 is mutated to any one of A, K, M, Q, R, S, and V; or
the amino acid at position 39 is mutated to any one of H, K, N, R, S, T, V, and Y;
the amino acid at position 40 is mutated to any one of A, C, D, E, G, H, K, M, N, Q, R, S, and Y; or
the amino acid at position 41 is mutated to any one of A, D, E, H, N, Q, S, and W; or
the amino acid at position 42 is mutated to any one of A, D, E, F, G, H, I, K, L, M, N, P, Q, R, S, T, V, W, and Y; or
the amino acid at position 43 is mutated to any one of A, G, I, K, N, R, S, T, V, and Y; or
the amino acid at position 44 is mutated to any one of A, G, and S; or
the amino acid at position 45 is mutated to any one of the following: A, D, F, G, H, K, L, M, N, Q, R, S, V, W, and Y; or
the amino acid at position 46 is mutated to any one of the following: A, D, E, F, H, I, K, L, M, N, P, Q, R, S, T, V, W, and Y; or
the amino acid at position 47 is mutated to any one of the following: I, L, M, Q, and V; or
the amino acid at position 48 is mutated to any one of the following: A, C, D, E, F, G, H, I, K, L, M, N, Q, R, S, T, V, W, and Y; or
the amino acid at position 49 is mutated to any one of the following: A, D, E, F, G, H, I, K, L, M, N, Q, R, S, T, V, W, and Y; or
the amino acid at position 50 is mutated to any one of the following: A, D, E, F, G, H, I, K, L, M, N, Q, R, S, T, V, W, and Y; or
the amino acid at position 51 is mutated to any one of A, C, G, I, L, M, S, T, and V; or
the amino acid at position 52 is mutated to any one of A, C, D, E, F, G, H, I, K, L, M, N, Q, R, S, T, V, W, and Y; or
the amino acid at position 53 is mutated to any one of A, C, D, E, F, G, H, I, K, L, M, N, Q, R, S, T, V, W, and Y; or
the amino acid at position 54 is mutated to any one of A, E, F, H, I, K, L, M, N, Q, R, S, T, V, W, and Y; or
the amino acid at position 55 is mutated to any one of F, I, L, M, S, and T.

Among these mutations, the following were selected: mutation with the amino acid at position 4 mutated from R to W; mutation with the amino acid at position 5 mutated from E to V, or I; mutation with the amino acid at position 6 mutated from L to F, or W; mutation with the amino acid at position 19 mutated from L to Q; mutation with the amino acid at position 24 mutated from L to M; mutation with the amino acid at position 32 mutated from L to V, or A; mutation with the amino acid at position 35 mutated from F to H, or Y; and mutation with the amino acid at position 48 mutated from F to R, L, or M. These mutations were then arranged and combined, and a new round of screening was conducted using the screening method described above. The concentration of biotinylated CD276 was gradually reduced from 100 nM to 1 nM. The binding miniprotein mutant-HM9 (SEQ ID No.: 2) was obtained after the final round of screening.

### Example 2: Expression and Purification of CD276_mb and HM9

The genes encoding CD276_mb and HM9 were cloned into a pET-28a(+) *E. coli* expression vector with a 6×His tag and thrombin cleavage site (SEQ ID No. 11: MGSSHHHHSSGLVPRGS) at the N-terminus. The cloned plasmid was transformed into *E. coli BL21(DE3)* (EC1002, WEIDIBio) and cultured in 1 L LB broth at 37°C and 220 RPM until the OD600 reached 0.6-0.8. IPTG (206-703-0, MACKLIN) was added to a final concentration of 250 µM, and the cells were cultured overnight at 37°C and 220 RPM. Cells were collected by centrifugation at 8000g for 5 minutes and resuspended in 25 ml protein buffer (25 mM Tris-HCl (pH 8.0) + 150 mM NaCl) and lysed by sonication for 10 minutes. Whole-cell lysates were separated by centrifugation at 14000 RPM for 30 minutes. The target protein was purified from the supernatant using Ni-NTA resin. Samples of whole-cell lysate, supernatant, flow-through, washing solution, and elution solution were sampled and run on a 15% SurePAGE gel (M00719, GenScript). The gel image is shown in Figure 2.

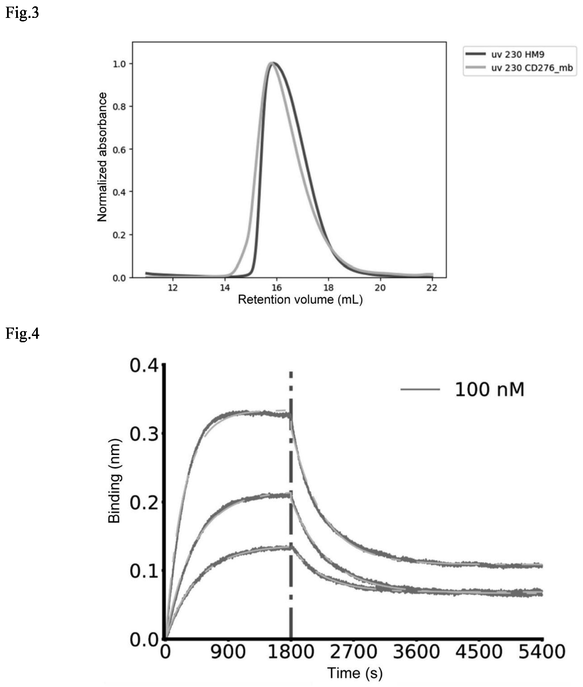

The target protein was further purified using molecular sieve chromatography on a Superdex 75 10/300 GL column (29148721, Cytiva) in PBS buffer (pH 8.0). The chromatogram is shown in Figure 3.

It can be seen from Figure 3 that both CD276_mb and HM9 were expressed at high levels in *E. coli BL21(DE3)*, could be purified by Ni-NTA resin, and both were in a monomeric form rather than in a polymeric form, indicating their stable properties.

### Example 3: Determination of the affinity between CD276_mb and CD276, and between HM9 and CD276 using BLI.

The concentrations of purified protein were determined using a Bradford Protein Assay Kit (P0006, Beyotime). The affinity of CD276_mb and HM9 for CD276 was determined using BLI experiments at concentrations of 100 nM, 50 nM, 25 nM, 12.5 nM, and 6.25 nM.

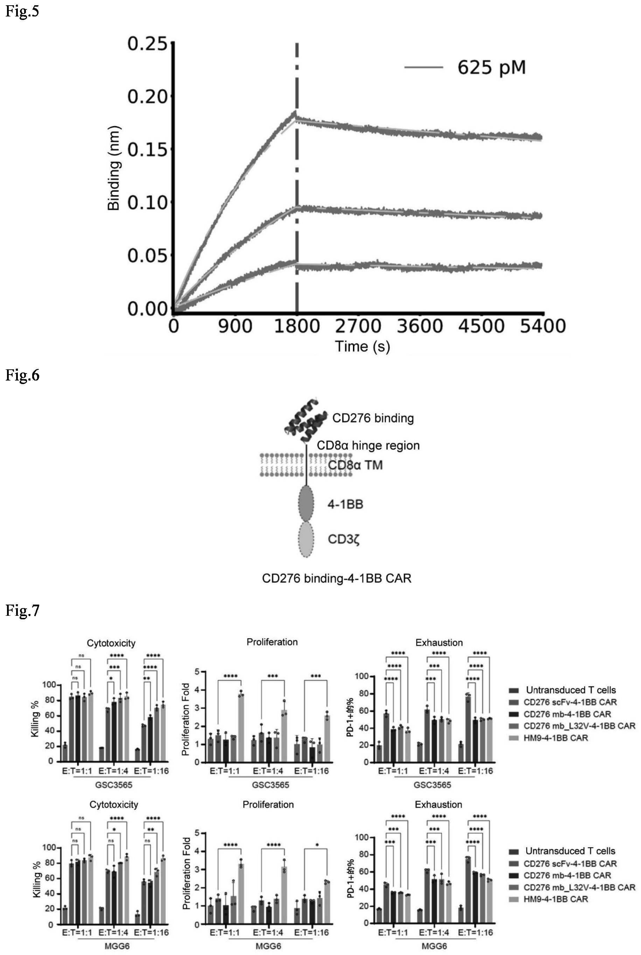

The results are shown in Figures 4 and 5. The figures show that the binding affinity of CD276_mb to CD276 after fitting was approximately 12.0 nM; the binding affinity of HM9 to CD276 after fitting was approximately 36.4 pM. This confirms that the HM9 obtained in the present application have strong binding activity for CD276.

**Example 4**: CD276_mb CAR-T design and determination of the killing, T cell proliferation, and exhaustion of HM9 CAR-T cells on different tumor cells using flow cytometry.

The HM9 sequence was integrated into the CAR structure, forming a complete HM9CAR coding sequence comprising CD8α signal peptide-HM9-CD8α hinge-CD8α transmembrane domain-4-1BB co-stimulatory domain-CD3ζ stimulatory domain. This HM9CAR sequence was cloned into the pCDH-CMV-MCS-EF1-copGFP (CD511B-1, systembio) vector. The structural schematic of the CAR (SEQ ID No.: 3) is shown in Figure 6. Wherein, the amino acid sequence of the signal peptide is SEQ ID No.: 4; the amino acid sequence of the hinge region is SEQ ID No.: 5; the amino acid sequence of the transmembrane domain is SEQ ID No.: 6; the amino acid sequence of the intracellular co-stimulatory signal transduction domain is SEQ ID No.: 7; and the amino acid sequence of the intracellular stimulatory signal transduction domain is SEQ ID No.: 8.

Simultaneously, different CD276_mb and CD276 scFv (clone 376.96, SEQ ID No.:12) were constructed into CAR structures in the same manner as experimental controls.

The constructed CAR plasmids were transfected into HEK293T (CRL-3216, ATCC) cells with psPAX2 (12260, Addgene) and pMD2.G (12259, Addgene), respectively, to produce lentiviruses. These lentiviruses were then used to infect T cells isolated from normal human PBMCs, which were then transformed into biologically functional CAR-T cells, namely HM9-4-1BB CAR-T cells.

HM9-4-1BB CAR-T cells were co-cultured with CD276-positive patient-derived glioblastoma cells GSC3565 and MGG4 at different effector (E)/target (T) ratios (1:1, 1:4, 1:16) for 48 hours. The remaining cells were analyzed by multicolor flow cytometry (CD3-APC (OKT3, BioLegend); PD-1-PE (EH12.2H7, BioLegend); Fixable Viability Dye eFluo^{TM} 780 (65-0865-18, Thermo)), and absolute cell population counting was performed using Precision Count Beads (424902, BioLegend). Killing % = number of tumor cells (target wells) / number of tumor cells (tumor culture wells only); proliferation fold = number of T cells (target wells) / number of T cells (T cell culture wells only); PD-1+ % = number of PD-1 positive cells / number of T cells. Flow cytometry was used to determine the tumor-killing ability, cell proliferation ability, and anti-exhaustion ability of HM9-4-1BB CAR-T and scFv-4-1BB CAR-T cells against different glioblastoma tumor cells, GSC3565 and MGG6. The results are shown in Figure 7.

As shown in Figure 7, compared to scFv-4-1BB CAR-T cells, the CAR-T cells constructed in the present application exhibit higher tumor-killing activity, lower exhaustion levels, and faster division and expansion capacity against different tumor cell types. Its significantly enhanced cell proliferation capacity and low exhaustion characteristics indicate that, compared to scFv-4-1BB CAR-T cells, HM9-4-1BB CAR-T cells can maintain a more prolonged state of normal activated cytotoxicity and perform tumor-killing functions when facing solid tumor challenges, avoiding premature exhaustion and T cell dysfunction.

### Example 5: Therapeutic Effect of HM9-4-1BB CAR-T Cells on Tumor-Bearing Mice

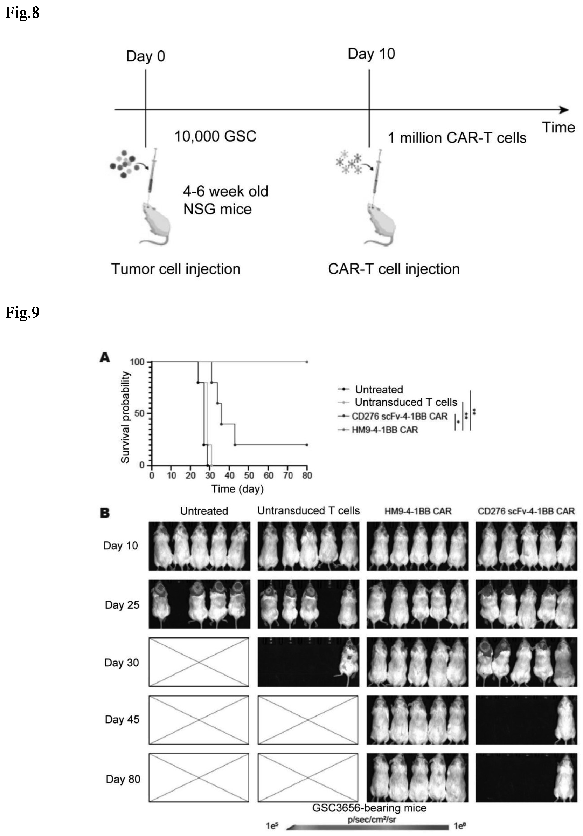

Patient-derived glioblastoma cells (10,000 cells/3 µL PBS) were injected intracranially into NOD/SCID/IL2Rg-/- (NSG) mice (005557, JAX Lab) using a stereotactic injection device at Bregma coordinates: x=+2.0 mm, y=-2.0 mm, z=-3.5 mm. Ten days after tumor implantation, the mice were injected with orthotopic CAR-T cell therapy (1,000,000 cells/3 µL PBS) for treatment. The flowchart is shown in Figure 8.

Tumor occurrence and development were recorded after injection, as well as the time points at which neurological symptoms appeared. Survival curves for tumor-bearing mice were plotted.

Results analysis is shown in Figure 9. The data in Figure 9 showed that mice treated with HM9-4-1BB CAR-T survived for more than 80 days and showed no detectable tumor signal generation. In contrast, mice treated with scFv-4-1BB CAR-T, while having prolonged survival, showed varying degrees of tumor occurrence. These results confirm that the HM9-4-1BB CAR-T cells constructed in the present application demonstrates significantly superior efficacy in tumor treatment compared to the scFv-4-1BB CAR-T cells in the prior art.

## Claims

1. A CD276 binding miniprotein or a mutant thereof,
wherein, the CD276 binding miniprotein has an amino acid sequence set forth in SEQ ID No.: 1, and
the mutant thereof is selected from:
i) a mutant with an amino acid sequence set forth in SEQ ID No.: 2;
ii) a single-site mutant of SEQ ID No.: 1,
with the amino acid at position 1 mutated to any one of A, D, E, F, G, H, I, L, M, N, P, R, T, V, W, and Y; or
with the amino acid at position 2 mutated to any one of A, D, E, F, G, H, K, L, M, N, P, Q, R, S, T, V, and W; or
with the amino acid at position 3 mutated to any one of A, D, E, F, G, H, I, K, L, M, N, P, Q, T, V, W, and Y; or
with the amino acid at position 4 mutated to any one of A, D, F, G, H, I, K, L, M, N, P, Q, R, S, T, V, W, and Y; or
with the amino acid at position 5 mutated to any one of A, D, E, H, I, N, Q, S, T, and V; or
with the amino acid at position 6 mutated to any one of F, L, M, W, and Y; or
with the amino acid at position 7 mutated to any one of A, D, E, F, G, H, I, K, L, M, N, Q, R, S, T, V, W, and Y; or
with the amino acid at position 8 mutated to any one of I, M, and V;
with the amino acid at position 9 is mutated to any one of F, H, I, L, and Y; or
with the amino acid at position 10 mutated to any one of A, D, E, F, H, I, L, M, Q, S, T, V, W, and Y; or
with the amino acid at position 11 mutated to any one of A, H, K, N, Q, R, S, T, V, and Y; or
with the amino acid at position 12 mutated to T; or
with the amino acid at position 13 mutated to any one of A, F, G, S, and T; or
with the amino acid at position 14 mutated to any one of A, E, F, G, H, I, K, L, M, N, Q, R, S, T, V, W, and Y; or
with the amino acid at position 15 mutated to any one of A, D, G, H, N, S, T, and V; or
with the amino acid at position 16 mutated to L; or
with the amino acid at position 17 mutated to any one of E, F, I, L, M, T, and V; or
with the amino acid at position 18 mutated to any one of A, H, K, N, Q, R, and T; or
with the amino acid at position 19 mutated to any one of A, F, G, H, I, L, M, N, Q, R, S, T, and V; or
with the amino acid at position 20 mutated to any one of A, D, E, G, H, K, N, Q, R, S, and T; or
with the amino acid at position 21 mutated to any one of D, E, K, N, and Q; or
with the amino acid at position 22 mutated to any one of A, F, H, K, L, M, N, Q, R, S, T, V, and Y; or
with the amino acid at position 23 mutated to any one of A, D, E, F, G, H, I, K, L, M, N, P, Q, R, S, T, and Y; or
with the amino acid at position 24 mutated to any one of L, M, V, and W; or
with the amino acid at position 25 mutated to any one of L, and M; or
with the amino acid at position 26 mutated to any one of A, C, D, E, F, H, I, K, L, M, N, P, Q, R, S, T, V, W, and Y; or
with the amino acid at position 27 mutated to any one of H, I, P, and V; or
with the amino acid at position 28 mutated to any one of A, C, I, L, M, S, and V; or
with the amino acid at position 29 mutated to any one of A, C, E, F, G, H, I, K, L, M, N, Q, R, S, T, V, W, and Y; or
with the amino acid at position 30 mutated to any one of A, D, E, F, G, H, K, M, N, Q, R, and W;
with the amino acid at position 31 mutated to any one of G, and S; or
with the amino acid at position 32 mutated to any one of A, C, F, I, L, M, S, T, V, and Y; or
with the amino acid at position 33 mutated to any one of A, C, D, E, G, H, I, K, L, M, N, Q, R, S, T, and Y; or
with the amino acid at position 34 mutated to any one of F, H, M, T, W, and Y; or
with the amino acid at position 35 mutated to any one of A, F, H, M, N, Q, and Y; or
with the amino acid at position 36 mutated to any one of C, D, E, F, H, I, K, L, M, N, R, S, V, W, and Y; or
with the amino acid at position 37 mutated to any one of A, D, E, F, G, H, I, K, L, M, N, Q, R, S, T, W, and Y; or
with The amino acid at position 38 mutated to any one of A, K, M, Q, R, S, and V; or
with the amino acid at position 39 mutated to any one of H, K, N, R, S, T, V, and Y;
with the amino acid at position 40 mutated to any one of A, C, D, E, G, H, K, M, N, Q, R, S, and Y; or
with the amino acid at position 41 mutated to any one of A, D, E, H, N, Q, S, and W; or
with the amino acid at position 42 mutated to any one of A, D, E, F, G, H, I, K, L, M, N, P, Q, R, S, T, V, W, and Y; or
with the amino acid at position 43 mutated to any one of A, G, I, K, N, R, S, T, V, and Y; or
with the amino acid at position 44 mutated to any one of A, G, and S; or
with the amino acid at position 45 mutated to any one of A, D, F, G, H, K, L, M, N, Q, R, S, V, W, and Y; or
with the amino acid at position 46 mutated to any one of A, D, E, F, H, I, K, L, M, N, P, Q, R, S, T, V, W, and Y; or
with the amino acid at position 47 mutated to any one of I, L, M, Q, and V; or
with the amino acid at position 48 mutated to any one of A, C, D, E, F, G, H, I, K, L, M, N, Q, R, S, T, V, W, and Y; or
with the amino acid at position 49 mutated to any one of A, D, E, F, G, H, I, K, L, M, N, Q, R, S, T, V, W, and Y; or
with the amino acid at position 50 mutated to any one of A, D, E, F, G, H, I, K, L, M, N, Q, R, S, T, V, W, and Y; or
with the amino acid at position 51 mutated to any one of A, C, G, I, L, M, S, T, and V; or
with the amino acid at position 52 mutated to any one of A, C, D, E, F, G, H, I, K, L, M, N, Q, R, S, T, V, W, and Y; or
with the amino acid at position 53 mutated to any one of A, C, D, E, F, G, H, I, K, L, M, N, Q, R, S, T, V, W, Y; or
with the amino acid at position 54 is mutated to any one of A, E, F, H, I, K, L, M, N, Q, R, S, T, V, W, and Y; or
with the amino acid at position 55 mutated to any one of F, I, L, M, S, and T, preferably, the CD276 binding miniprotein mutant has an amino acid sequence of SEQ ID No.: 2.

2. An isolated nucleic acid comprising a nucleic acid sequence encoding the CD276 binding miniprotein or the mutant thereof of claim 1.

3. A vector comprising the isolated nucleic acid of claim 2.

4. A chimeric antigen receptor comprising, from N-terminus to C-terminus:
(a) a signal peptide;
(b) an extracellular antigen-binding domain comprising the CD276 binding miniprotein or the mutant thereof of claim 1 or composed of the CD276 binding miniprotein or the mutant thereof of claim 1;
(c) a hinge region;
(d) a transmembrane domain;
(e) an intracellular co-stimulatory signal transduction domain; and
(f) an intracellular stimulatory signal transduction domain.

5. The chimeric antigen receptor according to claim 4, wherein, the signal peptide is derived from human CD8α and has an amino acid sequence of SEQ ID No.: 4; the hinge region is derived from human CD8α and has an amino acid sequence of SEQ ID No.: 5; the transmembrane domain is derived from human CD8α and has an amino acid sequence of SEQ ID No.: 6; the intracellular co-stimulatory signal transduction domain is derived from human 4-1BB and has an amino acid sequence of SEQ ID No.: 7; and the intracellular stimulatory signal transduction domain is derived from human CD3ζ and has an amino acid sequence of SEQ ID No.: 8,
preferably, the chimeric antigen receptor has an amino acid sequence of SEQ ID No.: 3.

6. An engineered immune effector cell comprising the chimeric antigen receptor of claim 4 or 5, preferably, the immune effector cell is a T cell, NK cell, or macrophage, further preferably, the engineered immune effector cell is a CAR-T cell.

7. A pharmaceutical composition comprising the aforementioned engineered immune effector cell and a pharmaceutically acceptable carrier.

8. An antibody comprising the CD276 binding miniprotein or the mutant thereof of claim 1.

9. Use of the CD276 binding miniprotein or the mutant thereof of claim 1 in the preparation of a monoclonal antibody, a bispecific antibody, an antibody-drug conjugate, or an engineered immune effector cell.

10. Use of the CD276 binding miniprotein or the mutant thereof of claim 1, the engineered immune effector cell of claim 6, the pharmaceutical composition of claim 7, or the antibody of claim 8 in the preparation of an antitumor drug,
preferably, the tumor is glioblastoma.
